Europäisches Patentamt

European Patent Office

Office européen des brevets

(19)

(11) Publication number: **0 605 721 A1**

## EUROPEAN PATENT APPLICATION
## published in accordance with Art. 158(3) EPC

(12)

(21) Application number: **92917991.9**

(22) Date of filing: **20.08.92**

(86) International application number:
**PCT/JP92/01054**

(87) International publication number:
**WO 93/03609 (04.03.93 93/06)**

(51) Int. Cl.5: **A01H  4/00**, A01C 1/00

(30) Priority: **20.08.91 JP 208250/91**

(43) Date of publication of application:
**13.07.94 Bulletin  94/28**

(84) Designated Contracting States:
**ES FR GB NL**

(71) Applicant: **KIRIN BEER KABUSHIKI KAISHA**
**26-1, Jingumae 6-chome**
**Shibuya-ku Tokyo 150(JP)**

(72) Inventor: **SAKAMOTO, Juyi, c/o Kirin Beer KK,**
**Shokubutsu KK**
**3377, Aza-Saruzuka**
**Oaza-Sotome,**
**Kitsuregawa-machi**
**Shioya-gun, Tochigi 329-14(JP)**
Inventor: **MASUDA, Yuko, Kabushiki Kaisha**
**Kirin City**
**K&K Building, 3rd Floor,**
**9-10, Dogenzaka 2-chome**
**Shibuya-ku, Tokyo 150(JP)**

(74) Representative: **Hansen, Bernd, Dr.**
**Dipl.-Chem. et al**
**Hoffmann, Eitle & Partner,**
**Patentanwälte,**
**Arabellastrasse 4**
**D-81925 München (DE)**

(54) **SELF-DEHISCENT ARTIFICIAL SEED.**

(57)  A self-dehiscent artificial seed characterized in that a plant propagation material which grows into a plant body is enclosed therein, that the tunic mainly comprises an alginic acid gel wherein part of complexing cations are replaced by non-complexing cations, that the surface of the tunic is hardened, and optionally that the tunic and/or the periphery thereof contain(s) an ion exchanger. A self-dehiscent artificial seed characterized in that a plant propagation material which grows into a plant body is enclosed therein, that the tunic mainly comprises an alginic acid gel complexed with complexing cations, that the surface of the tunic is hardened, and that the periphery of the tunic contains an ion exchanger. A self-dehiscent hydrogel globule mainly comprising an alginic acid gel wherein part of complexing cations are replaced by non-complexing cations, characterized in that the surface of the hydrogel globule is hardened or the inside and/or the periphery of the hydrogel globule contain(s) an ion exchanger. A self-dehiscent hydrogel globule mainly comprising an alginic acid gel complexed with complexing cations, characterized in that the surface of the the hydrogel globule is hardened and the periphery of the hydrogel contains an ion exchanger.

TECHNICAL FIELD

This invention relates to an artificial seed which comprises such plant propagules materials capable of growing into whole plants in future as seeds, somatic embryos derived from the tissue culture, etc. being encapsulated in an alginate gel.

TECHNICAL FIELD

Aqueous solutions of alginate-soluble salts are easily gelled by divalent or more metal ions and the like excluding mercuryion and magnesiumion. For example, since a large quantity of gel bead can be prepared by the liquid drop hardening method, alginate-soluble salts are suitable for a hydrogel matrix for artificial seeds. Artificial seeds using alginic acid gel salts are disclosed, for example, in U.S. Patent Nos. 4,562.633 and 4,779,376.

In the case of an artificial seed using alginate gel as the main matrix, there is a case where an expected germination rate cannot be obtained because of problems such as film formability, oxygen permeability, etc. of the alginate gel. The present inventors recognized that plant propagules capable of growing into plants, particularly plantlets derived from the tissue culture such as an somatic embryo and the like were favorably in quite adverse environments before and after seeding, that is, an anaerobic environment protecting an somatic embryo physically and at once suppressing germination and growth was favorable before seeding while an aerobic environment not inhibiting germination was favorable after seeding. Then, the present inventors studied the structure of such a gel bead as brings such environmental changes by transfiguring the structure dynamically before and after seeding and intensively studied a method for preparing the gel bead. As a result of it, we developed a self-dehiscent alginate gel bead (self-dehiscent gel bead) dependent upon washing with water and a treatment with a non-complexing cationic salt solution (Japanese Patent Application No. 254,403/1990).

The self-dehiscent gel bead is prepared by (1) thoroughly washing a gel bead after complexation in order to remove excess complexing cations, (2) treating the washed gel bead with a salt solution containing at last a monovalent cation such as a sodium ion, a potassium ion or the like, and then (3) washing thus treated gel bead with water. Thus obtained gel bead, when it is seeded in a humid environment, swells with the surrounding moisture and finally becomes unable to bear up its internal swelling to self-dehisce. In case of adopting such a self-dehiscing mechanism, a gel bead possesses an ability of protecting a plant propagule encapsulated therein when it is stored or transported. In addition, the gel bead self-dehisces with a small quantity of moisture after seeding to expose a part or the whole of the enclosed plant propagule material. The plant propagule material in the self-dehiscent gel bead vigorously germinates when physical inhibition of the growth at the time of germination, oxygen supply necessary for germination, etc. are improved.

The present inventors studied dehiscing conditions of the self-dehiscent gel bead in detail. The self-dehiscence of a gel bead is significantly influenced by the kind of soil, and self-dehiscence hardly took place in a certain kind of soil. In order to assume causes thereof, the inventors measured the electric conductivity of these soil eluates. In the case of soil showing high dehiscence rate of a gel bead such as vermiculite, Kanuma soil, etc., the electric conductivity was low (Nos. 1 - 4, Table 1). As compared with this, self-dehiscence degree of a gel bead was low in the case of soil containing humus substances. In the case of such soil, the electric conductivity of the eluate was high (Nos. 5-9, Table 1) and it was suggested this eluate contained highly concentrated salt in comparison with the aforementioned eluate. Then, the present inventors studied how the salt concentration of soil influenced on the dehiscence of a gel bead. As the result, it was observed that a gel bead was inhibited from the dehiscence by a salt of a certain concentration or more, and the salt concentration inhibiting the dehiscence depended upon the kind of salt (Table 2).

The present invention purposes to produce an artificial seed which self-dehisces even in soil in which a gel bead has been inhibited from self-dehiscence so far.

Table 1

| Relationship between electric conductivities of various soil eluates and self-dehiscence of gel bead | | | |
|---|---|---|---|
| Kind of Soil | Electric Conductivity [1] ($\mu$S) | | Self-dehiscence [2] (%) |
| | After 2 Hours | After 24 Hours | |
| 1. Horticubes (After washing) [3] | 25 | 66 | 100 |
| 2. Vermiculite | 66 | 21 | 74 |
| 3. Kanuma soil | 50 | 18 | 62 |
| 4. Perlite | 18 | 51 | 45 |
| 5. Soil for Raising Strawberry | 38 | 72 | 5 |
| 6. Kenbyo-kun [4] | 86 | 189 | 0 |
| 7. Soil for Horticultural Bed | 235 | 330 | 0 |
| 8. PRO-MIX A [5] | 385 | 405 | 0 |
| 9. Granular Culture Soil | 570 | 765 | 0 |

1) 5g each of Soil was suspended in 100ml of distilled water. After 2 hours and 24 hours, the electric conductivity of each eluate was measured.

2) After 24 hours from seeding the number of self-dehisced gel beads per 50 seeds were measured. The test was repeated twice, and the average values were given in the Table.

3) Manufactured by SMITHERS-OASIS, INC., U.S.A..

4) Trade name; manufactured by Nagano Federation of Economical Organization, JAPAN.

5) Manufacutred by PREMIER BRANDS, INC., CANADA.

Table 2

| Effect of kinds and concentrations of various ions on self-dehiscence of a gel bead[1] | | | |
|---|---|---|---|
| Kind of Salt | Concentration (mM) | Diameter [2] (mm) | Self-dehiscence (%) |
| (Tap Water) | - | 8.5 | 100 |
| (Deionized Water) | - | 8.9 | 100 |
| Calcium Chloride | 0.1 | 8.8 | 92 |
| | 0.3 | 7.3 | 4 |
| | 0.5 | 7.0 | 0 |
| | 1.0 | 6.5 | 0 |
| Aluminium Chloride | 0.1 | 8.9 | 100 |
| | 0.3 | 7.4 | 56 |
| | 0.5 | 6.8 | 0 |
| | 1.0 | 6.4 | 0 |
| Magnesium Chloride | 0.1 | 8.7 | 100 |
| | 0.3 | 8.4 | 76 |
| | 0.5 | 7.2 | 0 |
| | 1.0 | 7.1 | 0 |
| Sodium Chloride | 1.0 | 8.0 | 72 |
| | 2.0 | 7.6 | 24 |
| | 3.0 | 7.2 | 0 |
| | 5.0 | 7.0 | 0 |

1) Horticubes thoroughly washed with tap water were immersed in each solution, and gel bead were seeded on thus treated Horticubes. The diameters and the degrees of self-dehiscence of gel bead were measured after 24 hours (n = 50).

2) The diameter of a gel bead before seeding was 6.8mm.

EP 0 605 721 A1

DISCLOSURE OF THE INVENTION

The present invention provides a self-dehiscent artificial seed which is characterized by encapsulating a plant propagule capable of growing into a plant therein, the main matrix of its outer coat being composed of an alginate gel in which complexing cations are partially replaced by non-complexing cations, and the surface of said outer coat being hardened.

The present invention further provides a self-dehiscent artificial seed which is characterized by encapsulating a plant propagule capable of growing into a whole plant therein, the main matrix of its outer coat being composed of an alginate gel in which complexing cations are partially replaced by non-complexing cations, the surface of said outer coat being hardened, and ion exchangers being contained in the inside and the circumference of the outer coat.

The present invention further provides a self-dehiscent artificial seed which is characterized by encapsulating a plant propagule capable of growing into a whole plant therein, the main matrix of its outer coat being composed of an alginate gel in which complexing cations are partially replaced by non-complexing cations and ion exchangers being contained in the outer coat.

The present invention still further provides a self-dehiscent artificial seed which is characterized by encapsulating a plant propagule capable of growing into a plant therein, the main matrix of its outer coat being composed of an alginate gel complexed by complexing cations, the surface of said outer coat being hardened and ion-exchangers being contained in the circumference of the outer coat.

The present invention still further provides a self-dehiscent hydrogel bead which is characterized by being a hydrogel bead containing an alginate gel, in which complexing cations are partially replaced by non-complexing cations, as the main matrix, and the surface of said hydrogel bead being hardened.

The present invention still further provides a self-dehiscent hydrogel bead which is characterized by being a hydrogel bead containing an alginate gel, in which complexing cations are partially replaced by non-complexing cations, as the main matrix, the surface of said hydrogel bead being hardened and ion-exchangers being containing in the inside and/or the circumference of said hydrogel bead.

The present invention still further provides a self-dehiscent hydrogel bead which is characterized by being a hydrogel bead containing an alginate gel, in which complexing cations are partially replaced by non-complexing cations, as the main matrix, and ion-exchangers being contained in the inside of said hydrogel bead.

The present invention still further provides a self-dehiscent hydrogel bead which is characterized by being a hydrogel ball containing an alginate gel, in which complexing cations are partially replaced by non-complexing cations, as the main matrix, ion-exchangers being contained in the circumference of said hydrogel, and the surface of said hydrogel bead being hardened.

The plant propagule referred to in the present invention means a germ plasm capable of growing into a whole plant under a certain environment such as an somatic embryo, an adventitious shoot, a shoot primordium or the like which can be obtained by tissue culture besides a seed and a tuber. The plant referred to in the present invention means a higher plant such as a dicotyledon, a monocotyledon or the like. In addition, an artificial seed containing an alginate gel as the main matrix referred to in the present invention means a hydrogel bead in which a plant propagule is encapsulated and which contains alginate as the main matrix, and the outer coat of this hydrogel bead may contain hydrogel such as guar gum, carrageenan, etc., plant growth regulators for the plant propagule besides alginate as the main matrix. Furthermore, the self-dehiscent seed means an artificial seed donated with such a nature as a gel bead per se dehiscing after seeding to expose preferably a part of or the whole of a plant propagule. Self-dehiscent gel beads to be described hereinafter means hydrogel balls which is prepared according to the same method and similarly self-dehisce by the same treatment as said artificial seed except for not encapsulating a plant propagule therein. Incidentally, the hydrogel bead will be hereinafter referred to merely as a gel bead.

The alginate gel bead in which complexing cations are partially replaced by non-complexing cations referred to in the present invention means an alginate gel bead which swells with moisture and finally becomes unable to endure the internal pressure to self-dehisce under a humid environment. In case that a plant propagation material is not encapsulated, said gel bead will be hereinafter referred to as "a cation concentration-dependent self-dehiscent gel bead". This gel bead is prepared, for example, by (1) washing an alginate gel bead complexed by complexing cations with water for about 2 - 10 hours to removed the excess cations, (2) treating the washed gel bead with a solution containing 50mM - 2,000mM non-complexing monovalent cation such as sodium salt, potassium salt or the like for 5 minutes to 12 hours, and then (3) washing thus treated gel bead with water for about 10 minutes to 2 hours to remove the excess monovalent cations.

4

It was considered that the dehiscence inhibition of the cation concentration-dependent self-dehiscent gel bead was possibly caused by ions eluted from soil, as had been described in TECHNICAL BACKGROUND. In the course of studying a method for avoiding the same, the present inventors discovered that it was effective to use a substance adsorbing ions as a material of the gel bead. That is, it was found that the coexistence of a suitable ion exchanger with said gel bead came to prevent the seeded gel bead from the inhibition of swelling.

As an utilizable ion exchanger, the one adsorbing ions and having no or extremely small mobility in the gel matrix is preferable. In addition, it is effective to use not only a cation exchanger but also an anion exchanger independently or in combination. The ionic type of the ion exchanger is favorably $H^+$ for a cation exchanger and $OH^-$ for an anion exchanger. The ion exchanger includes various ion exchangers on cellolose, ion exchange resins, inorganic ion exchangers, ion blockers and polymer coagulants for industrial use, etc.

As examples of the ion exchangers on cellulose, cation exchanger on cellulose such as sulfoxyethyl cellulose, phosphomethyl cellulose, cellulose phosphate, carboxymethyl cellulose, etc. and anion exchanger on cellulose such as 2-hydroxyl-propyl aminocellulose, guanideethyl cellulose, triethyl aminocellulose, polyethylene iminocellulose, diethylaminoethyl cellulose, aminoethyl cellulose, ECTEOLA cellulose, aminobenzyl cellulose, etc. can be enumerated.

As examples of the ion exchanger on resins, styrene, methacrylic, acrylic and like cation exchanger on resins; polyamine, styrene and like anion exchanger on resins; chelate resins; etc. can be enumerated.

As examples of the inorganic ion exchangers, synthetic zeolites, various clay minerals, etc. can be enumerated.

In the present invention, these ion exchangers can be used as materials of a gel bead independently or in combination. Carboxymethyl cellulose, cross-linked polyacrylic acid, diethylaminoethyl cellulose, polyamines having tertiary amino residues, zeolite, etc. are favorable. However, the scope of the effect of the invention is nowise restricted to these.

These ion exchangers are used in a method by which they are enclosed in an alginate solution and in a method by which they are coated on the surface of a gel bead prepared according to the conventional methods. In case of only coating with an ion exchanger, it is preferred that the surface of a gel bead is previously hardened. In case of coating a gel bead having the unhardened surface with an ion exchanger, there are cases where gel beads have a low self-dehiscence rate or does not self-dehisce though they swell.

In case of preparing a gel bead by enclosing an ion exchanger in an alginate solution, a 0.5 - 3.5% (W/V) alginate solution in which a 0.1 - 20% (W/W), preferably 0.5 - 3.5% (W/W) ion exchanger is enclosed is used for the preparation of a cation concentration-dependent self-dehiscent gel bead. As described in Experimental Example 1, a gel ball, inside of which at last an ion exchanger had been enclosed, swelled noticeably in such soil as has a low eluting ion concentration, e.g., Kanuma soil or the like, and the increase in the self-dehiscence rate of a gel bead was also observed in many ion exchangers. Such acceleration of swelling and increase in the self-dehiscence rate of a gel bead also take place not only in the case of using a cation exchanger but also in the case of using an anion exchanger as a material of a gel bead. In addition, when a cation exchanger and an anion exchanger are used in combination, a gel bead dehisces effectively (Experimental Example 1 and Table 3).

In order to coat a gel bead with an ion exchanger, any method can be employed. For example, it can be easily attained by coating a cation concentration-dependent self-dehiscent gel bead prepared according to the aforementioned method with a powdery ion exchanger. Since a gel bead subjected to the powder coating treatment carries an ion exchanger on its outer surface, it is considered to be efficient for preventing the gel bead from the invasion of salts in soil which inhibits the self-dehiscence. In Example 1, a gel bead swelled without shrinking not only on Kanuma soil but also on a mixed culture soil of PRO-MIX A which had been suggested to have a high eluting ion concentration and Kanuma soil.

In addition, the present inventors intensively studied a method for more strongly self-dehiscing a gel bead swollen by the use of an ion exchanger. In the course of our study, we found the effectiveness of the surface hardening treatment of an alginate gel bead. As an effective surface hardener, a substance which reacts with alginate to form a fragile film having no or very small internal permeability is preferable. For example, not only various basic high molecular substances such as polyamino acids like $\alpha$-polylysine, $\gamma$-polylysine, etc.; basic polysacchrides like chitosan, etc.; bisbiguanides like chlorhexidine gluconate, etc. but such various metal ion salts such as an aluminium salt, an iron salt, a tin salt, etc., which have reactivity of film formation with alginate but have low permeability into the gel matrix, are effective.

Specifically, the surface hardening treatment comprises immersing a cation concentration-dependent self-dehiscent gel bead prepared according to the aforementioned method in the above hardener solution having a concentration of 0.05 - 30% (W/V), preferably of 0.2 - 5% (W/V), and washing thus treated gel bead with water for 5 seconds to 10 minutes. It goes without saying that this treatment is applicable not only to the above cation concentration-dependent self-dehiscent gel bead but also to a gel bead in which an ion exchanger is enclosed. Such surface hardening is very effective for converting the pressure owing to the swelling of a gel bead into high self-dehiscing ability of the gel bead. The combination of the surface hardening treatment with the enclosure of ion exchangers enables the self-dehiscence in soil considered to have a high eluting ion concentration (see Experimental Example 2). It is considered that high self-dehiscing ability of a cation concentration-dependent self-dehiscent gel bead in which at last an ion exchanger is enclosed and being subjected to surface hardening is brought because an ion exchanger adsorbs ions from soil to proceed the internal swelling of a gel bead on the one hand and the surface hardening inhibits the swelling near the surface on the other to thereby make the gel bead impossible to keep its original shape.

A cation concentration-dependent self-dehiscent gel bead to which an ion exchanger had not been applied but whose surface had been hardened was seeded in soil, and this gel bead self-dehisced. Accordingly, a cation concentration-dependent self-dehiscent gel bead self-dehisces on soil considered to have a high eluting ion concentration by subjecting the above surface hardening alone.

The relation of the enclosure of an ion exchanger, the coating of powder of an ion exchanger and the surface hardening treatment to the self-dehiscing rate of a cation concentration-dependent self-dehiscent gel bead in various soil is given in Table 5. That is, the surface powder coating of a gel bead undergone said self-dehiscing treatment with an ion exchanger, the enclosure of an ion exchanger in the gel bead and the surface hardening of the gel bead are independently effective for a certain soil. However, the combination of these enables self-dehiscence even in soil considered to have a high eluting ion concentration. The provision of artificial seeds which self-dehisces on various soil remarkably widens the applicability of self-dehiscent artificial seeds.

In addition, the combination of the enclosure of an ion exchanger with the surface hardening treatment disclosed in the present invention can give self-dehiscing ability also to an alginate bead which is not subjected to the cation concentration-dependent self-dehiscence treatment. In Experimental Example 5, a gel bead prepared by complexing a sodium alginate solution with complexing cations was washed with tap water, subjected to the surface hardening treatment, coated with powder of an ion exchanger and then seeded on mixed soil of Kanuma soil and PRO-MIX A [trade name; manufactured by PREMIER BRANDS INC. (Canada)]. Under the optimal combination of an ion exchanger and a treatment method, said gel bead self-dehisced 100%. In the case of this combination, even a gel bead which is not subjected to the a cation concentration-dependent self-dehiscence treatment can self-dehisce. According to the above combination, a manufacturing method of a self-dehiscent gel bead can be simplified.

[Experimental Examples]

Gel beads to be referred to in the following experimental examples are as same as that of the present invention, except that plant propagules are not encapsulated. When said gel beads are made to encapsulate plant propagules, the present artificial seed can be obtained.

Incidentally, concerning the indication of concentration in the following experimental examples, the quantities expressed in percentage mean W/V unless otherwise described particularly.

[Experimental Example 1]

Gel beads enclosing ion exchangers can be obtained by dropping a 1.5% sodium alginate (Duckalgin NSPH, manufactured by Kibun Food Chemifha K.K., JAPAN.) solution containing various 1% (W/W) ion exchangers in a 100mM calcium chloride solution to carry out complexation for 10 minutes, washing the gel beads with tap water for 5 minutes, immersed the washed the gel beads in 200mM potassium nitrate solutions for 90 minutes and finally washing thus treated gel beads with tap water for 40 minutes. A part of the obtained gel beads were seeded on Kanuma soil. The remaining gel beads (diameter, about 6 - 7mm) were drained, powder-coated with various ion exchangers (5mg/gel ball) and then seeded on Kanuma soil.

By enclosing cation exchangers and/or anion exchangers in gel beads or powder-coating the surface of gel beads with said ion exchangers, the average diameter of the gel beads increased about 11 - 40% before seeding (Table 3). In the case of gel beads in which both ion exchangers had been included, the self-dehiscence was accelerated in general. In the case of gel beads the surface of which had been powder-coated with an ion exchanger, the swelling of the gel beads was not always linked with the self-dehiscence (Table 3).

[Comparative Experimental Example 1]

A gel bead was prepared by dropping a 1.5% sodium alginate solution in a 100mM calcium chloride solution to carry out complexation for 10 minutes. Said gel bead was washed to desalt with tap water for 5 hours, immersed in a 200mM potassium nitrate solution, washed with water for 40 minutes and then seeded on Kanuma soil. The average diameter of the gel bead increased 13 - 16% before seeding. The self-dehiscence rate of this gel bead was 40 - 50% on the average (Table 3).

Table 3  Effect of various ion exchangers on the diameter

and the self-dehiscence of gel beads[1])

| Kind of Ion Exchanger | Enclosed in Gels | | Powder-coated the Surface | |
|---|---|---|---|---|
| | Diameter (mm) | Self-dehiscence (%) | Diameter (mm) | Self-dehiscence (%) |
| (Before seeding) | 6.8 | — | 7.1 | — |
| (Untreated) | 7.9 | 50 | 8.0 | 40 |
| Carboxymethyl cellulose[2] | 9.2 | 96 | 9.1 | 0 |
| Carboxymethyl cellulose[3] | 9.8 | 96 | 7.8 | 12 |
| Carboxymethyl cellulose[4] | 9.5 | 100 | 8.3 | 0 |
| Cellulose phosphate[5] | 8.1 | 56 | 9.6 | 0 |
| Polyacrylic acid (DOULITE ES468)[3] | 8.7 | 78 | 8.7 | 0 |
| DEAE-cellulose[2] | 7.9 | 26 | 8.9 | 0 |
| DEAE-cellulose (DE32)[4] | 7.8 | 48 | 9.3 | 60 |
| DEAE-cellulose (DE52)[4] | 8.9 | 86 | 8.9 | 32 |
| ECTEOLA-cellulose[5] | 8.1 | 48 | 8.5 | 12 |
| Polyamine (DOULITE A30B)[3] | 8.7 | 90 | 7.8 | 0 |
| Carboxymethyl cellulose[2] + DEAE-cellulose | 9.0 | 100 | 8.6 | 0 |
| Carboxymethyl cellulose[4] + DEAE-cellulose | 9.1 | 94 | 10.3 | 0 |
| Cellulose phosphate[5] + ECTEOLA-cellulose[5] | 8.1 | 36 | 9.1 | 44 |
| Polyacrylic acid[3] + poly-amine[3] | 9.0 | 100 | 8.6 | 0 |

1) Gel beads were seeded in Kanuma soil, and the diameters of and the self-dehiscence of the gel beads after 24 hours were measured (n=50).

2) Manufactured by Wako Pure Chemical K.K.

3) Manufactured by Serva, Inc.

4) Manufactured by Whatman, Inc.

5) Manufactured by Sigma Chemical Co.

[Experimental Example 2]

Gel beads prepared by dropping 1.5% sodium alginate solutions, in which carboxymethyl cellulose (hereinafter abbreviated to CM cellulose; CM32 manufactured by Whatman, Inc.) and diethylaminoethyl cellulose (hereinafter abbreviated to DEAE cellulose; DE32 manufactured by Whatman, Inc.) were so enclosed as to give a concentration of 3% (W/W) in all, in 100mM calcium chloride solutions to carry out complexation for 10 minutes. For comparison's sake, gel beads not enclosing cellulose ion exchangers were also prepared in the same manner as above. Both of these gel beads were washed with tap water for 5 hours, immersed in 200mM potassium nitrate solutions for 90 minutes, washed with water for 40 minutes, immersed in 0.5% poly(aluminium chloride) solutions, washed with water for 1 minute and then drained. Both of the drained gel beads were powder-coated (5mg/gel ball) with 2 kinds of ion exchangers mixed in various proportions, and then seeded on a mixed soil of Kanuma soil and PRO-MIX A [2 : 1 (V/V)]. As given in Table 4, the self-dehiscence rate was 100% in the case of gel beads enclosing and/or coated with a suitably combined ion exchangers.

[Comparative Experimental Example 2]

Gel beads, which had been prepared in the same manner as Experimental Example 2 except that an ion exchanger was not enclosed in, were seeded on a mixed soil of Kanuma soil and PRO-MIX A [2:1 (V/V)]. The self-dehiscence rate of said gel beads were 0% (see the combination of CE : AE = 0 : 0 in both cases of powder-coating and enclosure).

Table 4

| Application of ion exchangers for optimizing self-dehiscence of gel beads | | | | | | | | | | |
|---|---|---|---|---|---|---|---|---|---|---|
| | | Coating on Gel Surface | | | | | | | | |
| | CE : AE | 0 : 0 | 1 : 0 | 3 : 1 | 2 : 1 | 1 : 1 | 1 : 2 | 1 : 3 | 0 : 1 | |
| | CE : AE | Self-dehiscence (%)[1] | | | | | | | | |
| Enclosure in Gel Bead | 1 : 0 | 2 | 54 | 96 | 96 | 100 | 98 | 100 | 100 | |
| | 3 : 1 | 0 | 38 | 88 | 90 | 94 | 86 | 88 | 76 | |
| | 2 : 1 | 2 | 42 | 70 | 74 | 86 | 90 | 82 | 86 | |
| | 1 : 1 | 2 | 32 | 52 | 74 | 74 | 86 | 84 | 92 | |
| | 1 : 2 | 0 | 12 | 60 | 62 | 64 | 72 | 62 | 64 | |
| | 1 : 3 | 0 | 24 | 48 | 62 | 74 | 72 | 58 | 64 | |
| | 0 : 1 | 0 | 16 | 40 | 44 | 52 | 48 | 28 | 22 | |
| | 0 : 0 | 0 | 18 | 44 | 44 | 36 | 34 | 36 | 36 | |
| CE: Carboxymethyl cellulose (CM-cellulose) was used as a cation exchanger. | | | | | | | | | | |
| AE: Diethylaminoethyl cellulose (DEAE-cellulose) was used as an anion exchanger. | | | | | | | | | | |

1) The self-dehiscence of a gel bead was measured after 24 hours from seeding (n = 50).

[Experimental Example 3]

Various gel beads were prepared by combining the surface hardening treatment and the enclosure and the coating of ion exchangers according to the methods employed in Experimental Examples 1 and 2. The obtained gel beads were seeded on Horticubes LC-1 (trade name); manufactured by Smithers-Oasis, Inc. (U.S.A.)], Kanuma soil and mixed soil of Kanuma soil and PRO-MIX A [2:1 (V/V)]. The self-dehiscence of the seeded gel bead was measured after 24 hours from seeding (Table 5). The surface hardening and the powder-coating satisfactory self-dehiscence even in the case of mixed soil considered to have a high ion concentration. In addition, in the case of gel beads in which ion exchangers were enclosed, the dehiscence was accelerated in Kanuma soil, etc.

Table 5

| Effects of ion exchangers and surface hardening treatment on self-dehiscence rate of gel beads seeded on various soil | | | | | |
|---|---|---|---|---|---|
| Treatment | | | Self-dehiscence (%)[1] | | |
| Surface hardening | Enclosure | Powder coating | Horticube | Kanuma soil | PRO-MIX A + Kanuma soil [2] |
| O | O | O | 100 | 100 | 86 |
| O | O | X | 100 | 100 | 12 |
| O | X | O | 100 | 100 | 68 |
| O | X | X | 54 | 54 | 0 |
| X | O | O | 98 | 94 | 2 |
| X | O | X | 100 | 100 | 0 |
| X | X | O | 80 | 96 | 0 |
| X | X | X | 98 | 52 | 0 |

1) The self-dehiscence of a gel bead was measured after 24 hours from seeding (n = 50).
2) PRO-MIX A: Kanuma soil = 1 : 2 (V/V)

[Experimental Example 4]

Gel beads were prepared by dropping a 1.5% sodium alginate solution into a 100mM calcium chloride solution to carry out complexation for 10 minutes. The gel beads were washed with tap water for 5 hours, treated with a 200mM potassium nitrate solution for 90 minutes, washed with water for 40 minutes and then drained. Thus treated gel beads were immersed in 0.02, 0.1 and 0.5% poly(aluminium chloride) solutions for 10 seconds respectively, washed with water for 1 minutes, drained and then seeded on Kanuma soil.

As a result of it, the self-dehiscence rate of a gel bead which had not undergone the surface hardening (the control plot) was 52%, whereas those of gel beads which had undergone surface hardening treatment respectively with 0.02 and 0.1% poly(aluminium chloride) solutions were 100%. The self-dehiscence rate of a gel bead which had undergone the surface hardening treatment with a 0.5% poly(aluminium chloride) solution was 72%, which proved that excessive surface hardening lowered the dehiscence rate.

[Experimental Example 5]

Gel beads were prepared by dropping a 1.5% sodium alginate solution, in which CM cellulose and DEAE cellulose; were so enclosed as to give a concentration of 3% (W/W) in all, in a 100mM calcium chloride solutions to carry out complexation for 10 minutes. Gel beads enclosing no ion exchanger were also prepared in the same manner as above. These gel beads were washed with tap water for 5 hours, immersed in a 0.5% poly(aluminium chloride) solution for 10 seconds, washed with water for 1 minutes and then drained. Thus treated gel beads were powder-coated with ion exchangers exchangers in various proportions (16 mg/gel ball) and then seeded on mixed soil of Kanuma soil and PRO-MIX A [2 : 1 (V/V)]. The self-dehiscence rates of gel beads enclosing and/or coated with ion exchanger in suitable combination were all 100% (Table 6).

On the other hand, in the case of gel beads coated with ion exchangers in such combination as 5 mg/gel bead, their self-dehiscence rates were low. It became obvious that even a gel bead which did not undergo the cation concentration-dependant self-dehiscence treatment can self-dehisce by the use of suitable ion exchangers.

Table 6

| Application of ion exchangers for optimizing self-dehiscence of gel beads which did not undergo cation concentration-dependant self-dehiscence treatment | | | | | | | | | |
|---|---|---|---|---|---|---|---|---|---|
| | Powder-coating on Gel Surface (0.4g/25 beads) | | | | | | | | |
| | CE : AE | 0 : 0 | 1 : 0 | 3 : 1 | 2 : 1 | 1 : 1 | 1 : 2 | 1 : 3 | 0 : 1 |
| | CE : AE | Self-dehiscence (%)[1] | | | | | | | |
| Enclosure in Gel Bead | 1 : 0 | 0 | 100 | 100 | 100 | 72 | 20 | 8 | 0 |
| | 3 : 1 | 0 | 96 | 100 | 100 | 100 | 72 | 4 | 0 |
| | 2 : 1 | 0 | 88 | 100 | 100 | 100 | 103 | 40 | 0 |
| | 1 : 1 | 0 | 80 | 100 | 100 | 100 | 96 | 24 | 0 |
| | 1 : 2 | 0 | 8 | 103 | 100 | 100 | 92 | 48 | 0 |
| | 1 : 3 | 0 | 0 | 100 | 100 | 100 | 56 | 28 | 0 |
| | 0 : 1 | 0 | 0 | 96 | 100 | 100 | 60 | 36 | 0 |
| | 0 : 0 | 0 | 68 | 100 | 100 | 96 | 32 | 0 | 0 |

CE: Carboxymethyl-cellulose (CM-cellulose) was used as a cation exchanger.

AE: Diethylaminoethyl cellulose (uEAE-cellulose) was used as an anion exchanger.

1) The self-dehiscence of a gel bead was measured after 24 hours from seeding (n = 50).

BEST EMBODIMENT OF THE INVENTION:

[Example]

Somatic embryos deriverd from carrot (the major axis, 5mm) obtained according to the method of V. Vasil et al [Amer. J. Bot., vol. 69, p. 1441 (1982)], 3% (W/W) sucrose microcapsules (10% coating quantity; see Japanese Patent Application Laid-open No. 282,179/1989) and a 1.5% sodium alginate solution containing 30% CM cellulose were dropped in a 100mM calcium chloride solution to carry out complexation for 10 minutes, thereby preparing artificial seeds. These gel beads were washed with tap water for 5 hours, immersed in a 200mM potassium nitrate solution for 90 minutes and then washed with tap water for 40 minutes. Subsequently, these artificial seeds were immersed in a 0.5% poly(aluminium chloride) solution for 10 seconds, washed with water for 1 minutes, drained and then powder-coated (5 mg/gel bead) with mixed powder of GM-cellulose : DEAE-cellulose (1:3). Thus treated artificial seeds were seeded on mixed soil of Kanuma soil and PRO-MIX A [2 : 1 (V/V)]. All of the seeded artificial seeds self-dehisced, and the rooting from a somatic embryo was observed in 82% of the artificial seeds, and leaves of plantlets derived from the somatic embryos developed in 72%. The plantlets derived from the somatic embryos grew satisfactorily.

[Comparative Example]

Artificial seeds were prepared in the same manner as in the above example except for not enclosing CM cellulose and not subjecting to the poly(aluminium chloride) treatment, and then seeded on mixed soil of Kanuma soil and PRO-MIX A [2 : 1 (V/V)]. These seeds did not dehisce at all. In 32% of the artificial seeds, the rooting from somatic embryos was observed, and leaves of plantlets derived from the somatic embryos developed in 18%. However, 45% of the plantlets in which leaves had developed were inhibited from growth by gel sticking to them.

INDUSTRIAL UTILIZABILITY:

According to the present invention, it becomes possible to produce a self-dehiscent artificial seeds by combining various treatments in accordance with the kind of soil. This improved self-dehiscent artificial seed protects a plant propagule encapsulated therein before seeding on the one hand, and it self-dehisces to give a favorable environment accelerating the growth of the plant after seeding. Whereby, the germination ratio of an artificial seed is improved by leaps and bounds. Therefore, the present invention is very useful industrially.

**Claims**

1. A self-dehiscent artificial seed, characterized by encapsulating a plant propagation material capable of growing into a whole plant therein, the main matrix of the outer coat of said seed being composed of a alginate gel in which complexing cations is partially replaced by non-complexing cations, and the surface of said outer coat being hardened.

2. A self-dehiscent artificial seed according to Claim 1, wherein the complexing cations are calcium ions and the non-complexing cations are potassium ions or sodium ions.

3. A self-dehiscent artificial seed according to Claim 1, wherein the surface of the outer coat contains a surface hardener selected from polyamino acids, basic polysaccharides, bisbiguanides, basic high polymer substances, aluminium salts, iron salts and tin salts.

4. A self-dehiscent artificial seed according to Claim 1, wherein an ion exchanger is contained in the inside or the circumference of the outer coat.

5. A self-dehiscent artificial seed according to Claim 4, wherein the ion exchanger comprises at least 1 kind of ion exchanger selected from a group consists of cellulose ion exchangers, ion exchanger resins, inorganic ion exchangers, ion blockers and polymer coagulants.

6. A self-dehiscent artificial seed according to Claim 4, wherein the concentration of the ion exchanger is 0.1 - 20%.

EP 0 605 721 A1

7. A self-dehiscent artificial seed, characterized by containing a plant propagule capable of growing into a whole plant therein, the main matrix of the outer coat of said seed being composed of a alginate gel in which complexing cations is partially replaced by non-complexing cations, and said outer coat containing an ion exchanger.

8. A self-dehiscent artificial seed, characterized by containing a whole plant propagation material capable of growing into a plant therein, the main matrix of the outer coat of said seed being composed of a alginate gel in which complexing cations is partially replaced by non-complexing cations, the surface of said outer coat being hardened, and an ion exchanger being contained in the circumference of said outer coat.

9. A self-dehiscent hydrogel bead, characterized by being a hydrogel bead containing an alginate gel, in which complexing cations are partially replaced by non-complexing cations, as the main matrix, and the surface of said hydrogel bead being hardened.

10. A self-dehiscent hydrogel bead, characterized by being a hydrogel bead containing an alginate gel, in which complexing cations are partially replaced by non-complexing cations, as the main matrix, the surface of said hydrogel bead being hardened and ion-exchangers being contained in the inside and/or the circumference of said hydrogel bead.

11. A self-dehiscent hydrogel bead, characterized by being a hydrogel bead containing an alginate gel, in which complexing cations are partially replaced by non-complexing cations, as the main matrix, and ion-exchangers being contained in the inside of said hydrogel bead.

12. A self-dehiscent hydrogel bead, characterized by being a hydrogel bead containing an alginate gel, in which complexing cations are partially replaced by non-complexing cations, as the main ingredient, and ion-exchangers being contained in the circumference of said hydrogel bead, and the surface of said hydrogel bead being hardened.

13

# INTERNATIONAL SEARCH REPORT

International Application No PCT/JP92/01054

**I. CLASSIFICATION OF SUBJECT MATTER** (if several classification symbols apply, indicate all) 6

According to International Patent Classification (IPC) or to both National Classification and IPC

Int. Cl$^5$  A01H4/00, A01C1/00

**II. FIELDS SEARCHED**

| Minimum Documentation Searched 7 | |
| --- | --- |
| Classification System | Classification Symbols |
| IPC | A01H4/00, A01C1/00 |

Documentation Searched other than Minimum Documentation
to the Extent that such Documents are Included in the Fields Searched 8

| | |
| --- | --- |
| Jitsuyo Shinan Koho | 1975 – 1991 |
| Kokai Jitsuyo Shinan Koho | 1975 – 1991 |

**III. DOCUMENTS CONSIDERED TO BE RELEVANT** 9

| Category * | Citation of Document, 11 with indication, where appropriate, of the relevant passages 12 | Relevant to Claim No. 13 |
| --- | --- | --- |
| A | JP, A, 59-102308 (Plant Genetics, Inc.), June 13, 1984 (13. 06. 84), & US, A, 4562663, US, A, 4583320, US, A, 4715143, US, A, 4777762 | 1-12 |

* Special categories of cited documents: 10

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance; the claimed invention cannot be considered novel or cannot be considered to involve an inventive step

"Y"  document of particular relevance; the claimed invention cannot be considered to involve an inventive step when the document is combined with one or more other such documents, such combination being obvious to a person skilled in the art

"&"  document member of the same patent family

**IV. CERTIFICATION**

| Date of the Actual Completion of the International Search | Date of Mailing of this International Search Report |
| --- | --- |
| September 14, 1992 (14. 09. 92) | October 20, 1992 (20. 10. 92) |
| International Searching Authority | Signature of Authorized Officer |
| Japanese Patent Office | |

Form PCT/ISA/210 (second sheet) (January 1985)